# EUROPEAN PATENT APPLICATION

(11) **EP 4 060 020 A1**
(43) Date of publication of application: **21.09.2022**
(21) Application number: 20888506.1
(22) Date of filing: 11.11.2020
(51) Int. Cl.: C12M 3/00, C12N 5/07

(54) **CELL CULTURE CONTAINER, CELL CULTURE METHOD, AND METHOD FOR MANUFACTURING CELL CULTURE CONTAINER**

(30) Priority: 14.11.2019 JP 2019206248
(71) Applicant: SUMITOMO ELECTRIC INDUSTRIES, LTD., Osaka-shi, Osaka 541-0041 (JP)
(72) Inventor: MISU Hideyuki, Osaka-shi, Osaka 541-0041 (JP); SUGIYAMA Yoko, Osaka-shi, Osaka 541-0041 (JP); KIMURA Akinori, Osaka-shi, Osaka 541-0041 (JP); MOTOMURA Asako, Osaka-shi, Osaka 541-0041 (JP); SUGANUMA Hiroshi, Osaka-shi, Osaka 541-0041 (JP)
(74) Representative: Boult Wade Tennant LLP
(86) International application number: PCT/JP2020/042106
(87) International publication number: WO 2021/095776

(57) **Abstract**

A cell observation container includes a first container configured to accommodate a culture solution, and a second container having one or more wells that are able to be put in and taken out of the first container while retaining one or both of cells and cell masses cultured in the culture solution. One or more wells each include a bottom portion that is able to retain the culture solution, and a side wall that extends from a peripheral portion of the bottom portion. A liquid passage portion through which the culture solution accommodated in the first container is able to pass is provided in the side wall.

## Description

### Technical Field

The present disclosure relates to a cell culture container, a cell culture method, and a method for manufacturing a cell culture container. Priority is claimed on Japanese Patent Application No. 2019-206248, filed on November 14, 2019, the content of which is incorporated herein by reference.

### Background Art

As a container for culturing cells and cell masses (hereinafter referred to as "cell masses and the like"), a container in which a plurality of wells are arranged (so-called well plate) is known. Patent Literature 1 describes a cell culture container for culturing cell masses and the like in a culture solution. This cell culture container has a plurality of wells in which cell masses and the like to be cultured are retained.

### Citation List

### Patent Literature

[Patent Literature 1] PCT International Publication No. WO 2013-18377

### Summary of Invention

A cell observation container according to one embodiment of the present disclosure includes a first container configured to accommodate a culture solution, and a second container having one or more wells that are able to be put in and taken out of the first container while retaining one or both of cells and cell masses cultured in the culture solution. The one or more wells each include a bottom portion that is able to retain the culture solution, and a side wall that extends from a peripheral portion of the bottom portion. A liquid passage portion through which the culture solution accommodated in the first container is able to pass is provided in the side wall.

### Brief Description of Drawings

FIG. 1 is a cross-sectional view schematically showing a cell culture container according to one embodiment.
FIG. 2 is a cross-sectional view schematically showing a cell culture container according to one embodiment.
FIG. 3A is a plan view of the cell container shown in FIG. 1.
FIG. 3B is a cross-sectional view of a well of the cell container shown in FIG. 1 along the line IIIB-IIIB.
FIG. 4A is a cross-sectional view showing a mold used in a method of manufacturing a cell culture container according to one embodiment.
FIG. 4B is a view of an upper mold and a core mold shown in FIG. 4A when viewed from below.
FIG. 5A is a diagram illustrating one process of the method of manufacturing a cell culture container according to one embodiment.
FIG. 5B is a diagram showing a state after one process of the method of manufacturing a cell culture container shown in FIG. 5A.
FIG. 6 (a) portion to FIG. 6 (d) portion are schematic cross-sectional views showing changes in the culture state of cells and cell masses.
FIG. 7 is a cross-sectional view schematically illustrating a culture process of a cell culture method according to one embodiment.
FIG. 8 is a cross-sectional view schematically illustrating a culture process of the cell culture method according to one embodiment.
FIG. 9 is a cross-sectional view schematically illustrating an observation process of the cell culture method according to one embodiment.
FIG. 10 is a cross-sectional view schematically illustrating the observation process of the cell culture method according to one embodiment.
FIG. 11 (a) portion to FIG.11 (c) portion are cross-sectional views schematically illustrating each observation process in which the state of change in cell masses is observed.
FIG. 12A is a cross-sectional view schematically showing a cell culture container according to a modification example.
FIG. 12B is a cross-sectional view schematically showing the cell culture container according to the modification example shown in FIG. 12A.
FIG. 13A is a plan view schematically showing a cell container according to a modification example.
FIG. 13B is a plan view schematically showing a cell container according to a modification example.
FIG. 13C is a plan view schematically showing a cell container according to a modification example.
FIG. 13D is a plan view schematically showing a cell container according to a modification example.
FIG. 14A is a cross-sectional view schematically showing a well according to a modification example.
FIG. 14B is a cross-sectional view schematically showing a well according to a modification example.
FIG. 15A is a perspective view schematically showing a liquid passage portion according to a modification example.
FIG. 15B is a perspective view schematically showing a liquid passage portion according to a modification example.
FIG. 15C is a perspective view schematically showing a liquid passage portion according to a modification example.
FIG. 16 is a cross-sectional view schematically showing a liquid passage portion according to a modification example.
FIG. 17A is a cross-sectional view showing a mold used in a method of manufacturing a cell culture container according to a modification example.
FIG. 17B is a cross-sectional view of the mold shown in FIG. 17A along the line XVIIB-XVIIB.
FIG. 18 is a diagram showing some wells after molding by the method of manufacturing a cell culture container according to a modification example.
FIG. 19A is a diagram illustrating one process of a method of manufacturing a cell culture container according to a modification example.
FIG. 19B is a diagram showing a state after one process of the method of manufacturing a cell culture container shown in FIG. 19A.
FIG. 20A is a diagram showing one process of a method of manufacturing a cell culture container according to a modification example.
FIG. 20B is a diagram showing a state after one process of the method of manufacturing a cell culture container shown in FIG. 20A.
FIG. 21 is a diagram schematically showing a well according to another modification example.
FIG. 22 is a diagram schematically showing a well according to another modification example.
FIG. 23 is a diagram schematically showing a well according to another modification example.
FIG. 24 is a diagram schematically showing a well according to another modification example.
FIG. 25 is a diagram schematically showing a well according to another modification example.
FIG. 26 is a diagram schematically showing a well according to another modification example.
FIG. 27 is a diagram schematically showing a well according to another modification example.
FIG. 28 is a diagram schematically showing a well according to another modification example.
FIG. 29 is a diagram schematically showing a cell culture container according to another modification example.

### Description of Embodiments

### [Problem to be solved by the present disclosure]

When cell masses and the like are cultured using a cell culture container, the inside of the well is filled with a culture solution for culturing cell masses and the like. When cell masses and the like are cultured, in order to prevent cell masses and the like from drying out due to evaporation of the culture solution, it is required that a predetermined amount of the culture solution or more be present inside the well. However, if it is desired to optically observe cell masses in the culture procedure, the amount of light transmitted for observing the well may not be sufficiently obtained due to light absorption of the culture solution. In such a case, it is necessary to reduce the amount of the culture solution inside the well. In order to reduce the amount of the culture solution, for example, it is conceivable to manually remove the culture solution inside the well with a syringe or the like, which consumes time and effort.

### [Advantageous effects of the present disclosure]

According to the present disclosure, it is possible to efficiently observe the culture state of cell masses in a procedure of culturing cell masses.

### [Description of embodiments of the present disclosure]

First, details of embodiments of the present disclosure will be listed and described. A cell culture container according to one embodiment includes a first container configured to accommodate a culture solution and a second container having one or more wells that can be put in and taken out of the first container while retaining one or both of cells and cell masses cultured in the culture solution. One or more wells each has a bottom portion that can retain the culture solution and side walls that extend from a peripheral portion of the bottom portion. In the side wall, a liquid passage portion through which the culture solution accommodated in the first container can pass is provided.

In the cell culture container, the culture solution is put in and taken out of the well through the liquid passage portion provided in the side wall. Thus, the well can be filled with the culture solution simply by inserting the well into the first container in which the culture solution is accommodated. On the other hand, when the culture state of cell masses is observed, only some of the culture solution in the well can be easily discharged by simply ejecting the well from the first container while the culture solution is retained in the bottom portion. Therefore, it is possible to efficiently observe the culture state of cell masses in a procedure of culturing cells and cell masses.

In the cell culture container according to one embodiment, the liquid passage portion may be one or more holes that penetrate the side wall. The hole penetrating the side wall can realize a configuration that allows the culture solution to pass through.

In the cell culture container according to one embodiment, the material forming one or more wells may be polystyrene. Since polystyrene has a property of not easily adhering to cell masses and the like, it is suitable for culturing cell masses and the like. When the well is formed of polystyrene, for example, the cultured cell masses and the like can be easily removed from the well.

In the cell culture container according to one embodiment, the liquid passage portion is a region of at least a part of the side wall, and this region may be formed of a porous polymer material that allows the culture solution to pass through. In this case, the region of the side wall formed of a porous polymer material can realize a configuration that allows the culture solution to pass through.

In the cell culture container according to one embodiment, one or more wells may be a plurality of wells connected to each other. In this case, the heights from the lower ends of the bottom portions of the plurality of wells to the lower ends of the liquid passage portions may be constant. For example, in a configuration having a plurality of wells, when the culture state of cell masses is observed, if the culture solution in the well is manually removed with a syringe or the like, it is difficult to keep the amount of the culture solution remaining in the well constant. In addition, as the number of wells increases, time and effort increase. On the other hand, in the cell culture container according to one embodiment of the present disclosure, according to the configuration in which the heights from the lower ends of the bottom portions of the plurality of wells to the lower ends of the liquid passage portions are constant, by simply ejecting the well from the first container, it is possible to easily discharge only some of the culture solution in the well while a certain amount of the culture solution is retained in each bottom portion of each of the plurality of wells. Thereby, it is possible to efficiently observe the culture state of cell masses and the like accommodated in each well under the same conditions. Here, the term "constant" used here means not only a case in which the heights from the lower ends of the bottom portions to the lower ends of the liquid passage portions match, but also a case in which the height is within a range of ±10% with respect to the average height of the heights.

In the cell culture container according to one embodiment, the first container may have a plurality of accommodating spaces that some of the plurality of wells can be put in and taken out of, and one or more partition walls that separate the plurality of accommodating spaces from each other. In this case, since the plurality of accommodating spaces are water-tightly partitioned, the culture solution does not enter the well accommodated in each accommodating space from other accommodating spaces. Thus, it is possible to avoid contamination caused by the culture solution accommodated in the other accommodating spaces.

In the cell culture container according to one embodiment, the height from the lower end of the bottom portion to the lower end of the liquid passage portion may be 1.0 mm or more and 5.0 mm or less. When the liquid passage portion is positioned in such a range with respect to the bottom portion that can retain the culture solution, even if the culture solutions is discharged through the liquid passage portion when cell masses and the like are observed, the cell masses and the like can be prevented from drying out, and an observation operation can be easily performed. More specifically, when the height to the lower end of the liquid passage portion is 1.0 mm or more, even if cell masses and the like are observed, the cell masses and the like retained in the bottom portion can be more reliably immersed in the culture solution. In addition, if the height to the lower end of the liquid passage portion is 5.0 mm or less, when cell masses and the like are observed, it is possible to perform discharge more easily so that the amount of the culture solution that interferes with observation is more appropriate.

In the cell culture container according to one embodiment, one or more wells may each further include a guide member that guides a flow of the culture solution discharged from the liquid passage portion. If the well is lifted and an unnecessary culture solution is discharged from the well when the culture state of cell masses is observed, liquid dripping in which the discharged culture solution adheres to the lower part of the well occurs, which can interfere with observation of the cultured cell masses. However, when the cell culture container includes a guide member that prevents liquid dripping, it is possible to efficiently observe cell masses. Here, when a guide member is provided, the guide member is provided on the outer circumferential surface of each of one or more wells, and in a region closer to the lower end of the bottom portion than the liquid passage portion, and may extend in the opposite direction to the liquid passage portion. In this case, the guide member can be realized with a simple configuration.

A cell culture method according to one embodiment includes preparing any of the above cell culture containers, immersing cells retained in one or more wells in the culture solution accommodated in the first container to culture cells and cell masses obtained by aggregating cells, and observing the cell masses. The observing of the cell masses includes removing the second container from the first container, and the cell masses in the one or more wells may be observed when some of the culture solution is discharged from the one or more wells through the liquid passage portion. In this case, in the observing, some of the culture solution can be easily discharged from the well. Since cell masses in the well are observed when some of the culture solution is discharged, it is possible to prevent the culture solution from interfering with observation of cell masses. Thus, in the culturing of the cell masses, it is possible to efficiently observe the culture state of cell masses.

In the cell culture method according to one embodiment, the observing of the cell masses may include removing a liquid adhered to the outer circumference of the bottom portion of the second container after the second container is removed from the first container. In this case, it is possible to prevent the culture solution adhered to the outer circumference of the second container from interfering with observation of cell masses. Thus, in the culturing of the cell masses, it is possible to observe the culture state of cell masses more efficiently.

A method of manufacturing a cell culture container according to one embodiment is a method of manufacturing the cell culture container, including manufacturing a second container by molding. The manufacturing includes preparing a mold including an upper mold having a projection part having a shape corresponding to an outer shape of each of one or more wells, a lower mold that is arranged facing the projection part and has a recess part having a shape corresponding to an outer shape of each of the one or more wells, and a core mold that penetrates the projection part and the recess part, and curing a resin material poured into the mold to mold the second container including the one or more wells, and a hole as the liquid passage portion may be formed with the core mold. According to this method, since a hole as a liquid passage portion is formed in the well of the second container manufactured by molding, a cell culture container having the above configuration can be easily formed. Thus, when the manufactured cell culture container is used, in the culturing of the cell masses, it is possible to efficiently observe the culture state of cell masses.

A method of manufacturing a cell culture container according to another embodiment is a method of manufacturing the above cell culture container, including manufacturing a second container by molding. The manufacturing includes preparing a mold including an upper mold having a projection part having a shape corresponding to an outer shape of each of one or more wells and a lower mold that is arranged facing the projection part and has a recess part having a shape corresponding to an outer shape of each of one or more wells, and curing a resin material poured into the mold to mold the second container including one or more wells. The upper mold may have a protrusion that extends in a vertical direction, projects from the projection part, and is in contact with the recess part, and the protrusions may form a slit as the liquid passage portion. According to this method, since a slit as the liquid passage portion is formed in the well of the second container manufactured by molding, a cell culture container having the above configuration can be easily formed. Thus, when the manufactured cell culture container is used, in the culturing of the cell masses, it is possible to efficiently observe the culture state of cell masses.

A method of manufacturing a cell culture container according to still another embodiment is a method of manufacturing the above cell culture container, and the method may include molding a molded product exhibiting an outer shape of the one or more wells by molding and forming a hole as the liquid passage portion in the molded product by laser emission. According to this method, since a hole as a liquid passage portion is formed by laser emission in the manufactured well, a cell culture container having the above configuration can be easily formed. Therefore, when the manufactured cell culture container is used, in the culturing of the cell masses, it is possible to efficiently observe the culture state of cell masses.

### [Details of embodiments of the present disclosure]

Specific examples of a cell culture container, a cell culture method, and a method of manufacturing a cell culture container according to the present disclosure will be described below with reference to the drawings. Here, the present invention is not limited to these examples, but is indicated by the scope of the claims, and is intended to include meanings equivalent to the scope of the claims and all modifications within the scope of the claims. In the following description, the same elements or elements having the same function are denoted with the same reference numerals and redundant descriptions may be omitted.

### (Configuration of cell culture container)

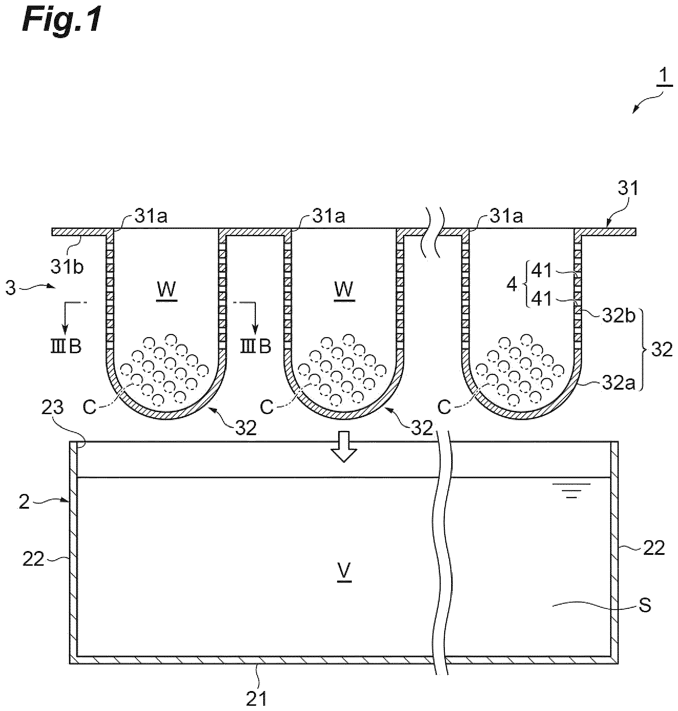

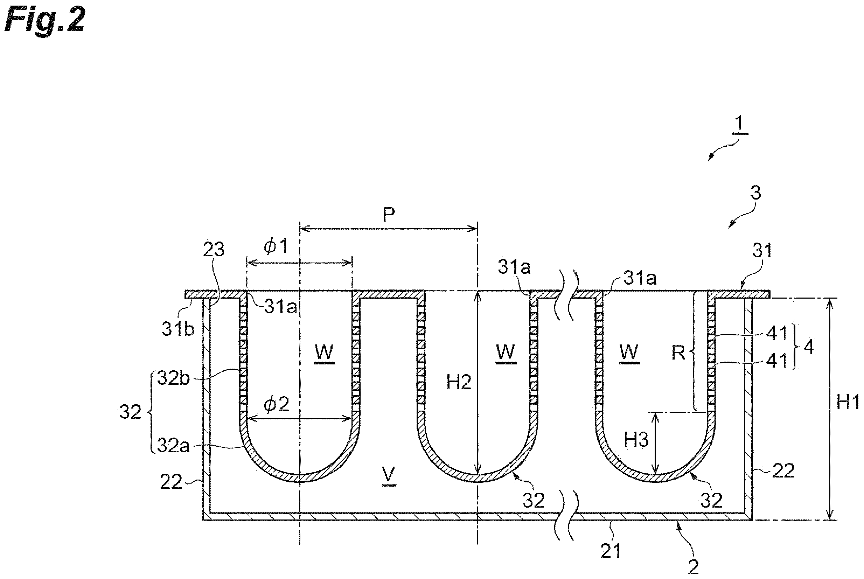

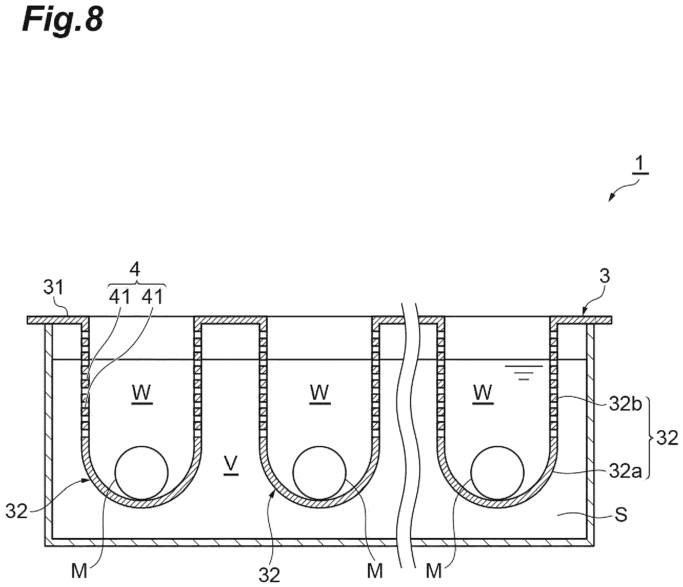

FIG. 1 and FIG. 2 are cross-sectional views schematically showing a cell culture container 1 according to one embodiment. The cell culture container 1 is used when cells C and cell masses M (refer to FIG. 8) are cultured using a culture solution S. In FIG. 1, cells C are indicated by an imaginary line. Specifically, the cell culture container 1 is a container in which cells C are aggregated to obtain cell masses M in a desired state in the culture solution S. The cell culture container 1 includes a culture solution container 2 (first container) and a cell container 3 (second container).

The culture solution container 2 is a container (dish) for accommodating the culture solution S. The culture solution container 2 is made of, for example, a resin. The resin material forming the culture solution container 2 is preferably, for example, polystyrene, but may be another resin, or may be glass or a metal. The inner surface of the culture solution container 2 may be subjected to a surface treatment in which coating with fluorine or the like is performed, or may be subjected to a surface treatment in which chemical resistance according to properties of the culture solution S is obtained. Here, the culture solution container 2 may not be formed of a transparent material. The shape of the culture solution container 2 is a substantially rectangular shape in a plan view. The culture solution container 2 includes a rectangular flat bottom plate 21 and four side walls 22. The bottom plate 21 extends so that it spreads along the horizontal plane when the culture solution S is accommodated (in the state shown in FIG. 1). The thickness of the bottom plate 21 is, for example, 1 mm. The length of the bottom plate 21 in the longitudinal direction is, for example, 120 mm or more and 132 mm or less. The length of the bottom plate 21 in the lateral direction is, for example, 80 mm or more and 90 mm or less.

The four side walls 22 stand along four edges of the bottom plate 21. Each side wall 22 extends upward from each edge of the bottom plate 21. The thickness of the side wall 22 is, for example, 1 mm. A height H1 of the side wall 22 is, for example, 14.5 mm. The bottom plate 21 and the four side walls 22 define an accommodating space V for accommodating the culture solution S. In the present embodiment, the culture solution container 2 has only one accommodating space V. The culture solution container 2 has an opening 23 that opens the accommodating space V on the opposite side to the bottom plate 21. When the bottom plate 21 is disposed along the horizontal plane, the opening 23 opens the accommodating space V upward.

The cell container 3 is, for example, a resin well plate. In the present embodiment, the cell container 3 is formed of a material that allows transmission with respect to a light source for observation (for example, a light source L to be described below), and may be made transparent to visible light. The material forming the cell container 3 is preferably, for example, polystyrene, but may be polycarbonate or glass. The cell container 3 has a plate-like plate 31 and a plurality of (in the present embodiment, e.g. ninety six) wells 32 provided on the plate 31. As will be described below, the well 32 includes a bottom portion 32a and a side wall 32b. In the cell container 3, the bottom portion 32a of the well 32 may be formed of a material that allows transmission with respect to a light source for observation, and the other parts (for example, the side wall 32b) may be formed of a material that does not allow transmission with respect to the light source. The inner surface of the well 32 of the cell container 3 may be subjected to a surface treatment to prevent cells C from adhering or a chemical-resistant surface treatment. The region of the cell container 3 other than the bottom portion 32a (the plate 31 or the side wall 32b) may be formed of a material different from the material of the bottom portion 32a, that is, a material that does not allow transmission with respect to a light source, for example, may be formed of an acrylic resin, a fluororesin (e.g. PTFE) or a metal, or may be formed of a fiber such as a non-woven fabric. The region of the well 32 other than the inner surface of the bottom portion 32a may be subjected to a surface treatment that makes it difficult for cells to adhere or a chemical-resistant surface treatment. The cell container 3 may be formed by appropriately combining the above plurality of materials.

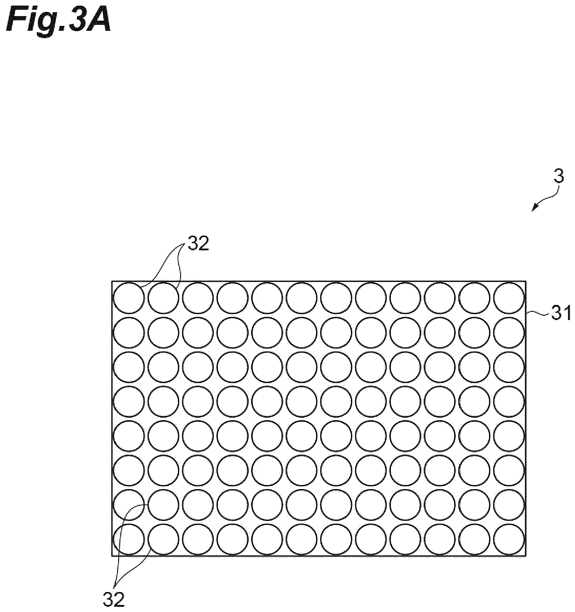

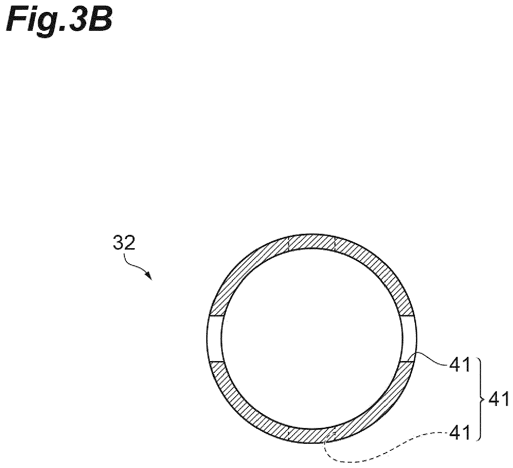

The cell container 3 having such a configuration is separate from the culture solution container 2. FIG. 3A is a plan view of the cell container 3 shown in FIG. 1. FIG. 3B is a cross-sectional view of the well 32 of the cell container 3 shown in FIG. 1 along the line IIIB-IIIB. As shown in FIG. 1 to FIG. 3A, in a plan view, the outer shape of the plate 31 is a substantially rectangular shape that is a size larger than the bottom plate 21. The thickness of the plate 31 is, for example, 1 mm. In the plate 31, a plurality of (the same number as the wells 32, in the present embodiment, e.g. ninety six) connection holes 31a which penetrate the plate 31 in the thickness direction to be connected with the wells 32 are arranged. The inner diameter ϕ1 of the connection hole 31a is, for example, 6.0 mm. The plate 31 connects the plurality of wells 32 to each other. The plate 31 has an outer shape larger than the opening 23 of the culture solution container 2. When the cell container 3 is installed in the culture solution container 2, the plate 31 comes into contact with the edge of the opening 23 of the culture solution container 2, and thus positioning of each well 32 in the culture solution container 2 is performed.

The well 32 is a container for retaining cells C and cell masses M cultured in the culture solution S. The thickness of the well 32 is, for example, 1 mm. The plurality of wells 32 can be put in and taken out of the accommodating space V of the culture solution container 2. In the present embodiment, all wells 32 can be put in and taken out of one accommodating space V. Each of the plurality of wells 32 is connected to the connection hole 31a. The plurality of wells 32 are arranged on one main surface 31b of the plate 31. In the present embodiment, the cell container 3 has wells 32 arranged in 12 columns in the longitudinal direction of the plate 31 and 8 rows of wells 32 in the lateral direction of the plate 31. The pitch P between two adjacent wells 32 is constant (for example, 9.0 mm). The plurality of wells 32 are collectively arranged in the accommodating space V by moving the plate 31 close to the culture solution container 2, and collectively ejected from the accommodating space V by separating the plate 31 from the culture solution container 2.

The well 32 has the bottom portion 32a and the side wall 32b connecting the bottom portion 32a with the plate 31. The bottom portion 32a has a recess shape that is recessed away from the plate 31 and can retain the culture solution S. The outer shape of the bottom portion 32a is, for example, a hemispherical shape. The inner diameter ϕ2 of the bottom portion 32a is, for example, 5.2 mm. The side wall 32b is a tubular part extending from the peripheral portion of the bottom portion 32a to the connection hole 31a of the plate 31. The bottom portion 32a and the side wall 32b define a culture space W for retaining cultured cells C and cell masses M. A height H2 of the culture space W is smaller than the height H1 of the side wall 22. The height H2 is, for example, 10.5 mm. The end of the side wall 32b on the side opposite to the bottom plate 21 communicates with the connection hole 31a of the plate 31, and the culture space W is open to the external space through the connection hole 31a.

A liquid passage portion 4 through which the culture solution S can pass is provided in the side wall 32b. The liquid passage portion 4 has a configuration that at least does not allow cell masses M to pass through. The liquid passage portion 4 may have a configuration that does not allow cells C to pass through. In the present embodiment, the liquid passage portion 4 is one or a plurality of holes 41. The number of holes 41 may be set arbitrarily. In the present embodiment, an example in which the plurality of holes 41 are provided will be described. The holes 41 penetrate the side wall 32b in the thickness direction of the side wall 32b. The holes 41 may penetrate the side wall 32b in a direction inclined with respect to the thickness direction of the side wall 32b. The holes 41 communicates with the culture space W and the external space. Thereby, the holes 41 allows the culture solution S to pass through. The shape of the hole 41 is, for example, a circular shape when viewed in the penetration direction (that is, the thickness direction of the side wall 32b). The in-plane size of the hole 41 intersecting the penetration direction is a size at which cell masses M do not pass through, for example, a diameter of 0.01 mm or more, and a diameter of 6.0 mm or less. Alternatively, the in-plane size of the hole 41 intersecting the penetration direction may be a diameter of 3.0 mm or less and a diameter of 1.0 mm or less. In the present embodiment, the in-plane size (diameter) of the hole 41 intersecting the penetration direction is, for example, 1.0 mm.

The liquid passage portion 4 is provided at a position separated from the tip (lower end) of the bottom portion 32a by a predetermined height H3 or more. In this manner, when the liquid passage portion 4 is provided at a position away from the bottom portion 32a, it is possible to prevent cell masses M from drying out during observation. The height H3 from the lower end of the bottom portion 32a to the lower end of the liquid passage portion 4 may be 1.0 mm or more, for example, when the diameter of the cultured cell masses M is 1 mm or less, and the height H3 may be 5.0 mm or less. When the diameter of the cultured cell masses M is 0.1 mm or more and 2 mm or less, the height H3 may be 1.0 mm or more, and the height H3 may be 4.0 mm or less. In a pattern of the relationship between the diameter of the cell masses M and the height H3 of the water surface of the culture solution, for example, when the diameter of the cell masses M is 1 mm or less, the height H3 may be 2 mm, and when the diameter of the cell masses M is 1 mm or more and 2 mm or less, the height H3 may be 3 mm or more and 4 mm or less. The height H3 is preferably as low as possible so that the cell masses M are not exposed from the water surface of the culture solution for preventing drying and the culture solution does not interfere with observation, and the height H3 may not be within the above range. When the top surface of the cell masses M and the water surface of the culture solution are too close to each other, the water surface directly above the cell masses M becomes projected due to the influence of surface tension, and light for observation may not be guided in a straight line to a microscope and may interfere with observation. Thus, it is preferable to secure a distance of at least 1 mm or more or 2 mm or more as a distance from the upper part of the cell masses M to the water surface of the culture solution, for example. The liquid passage portion 4 may be provided over a predetermined range R extending toward the plate 31 from a position separated from the lower end of the bottom portion 32a by a height H3. In the present embodiment, the range R is the entire region of the side wall 32b. The range R may be a part of the region of the side wall 32b. In the present embodiment, the plurality of holes 41 are distributed and provided in the range R. The height H3 is constant among the plurality of wells 32. In other words, among the plurality of wells 32, the heights H3 of the holes 41 positioned at the lowermost ends are equal to each other. The term "constant" used here means not only a case in which the heights H3 from the lower ends of the bottom portions 32a to the lower ends of the liquid passage portions 4 match, but also a case in which the height is within a range of ±10% with respect to the average height of the heights H3. The plurality of holes 41 other than the holes 41 positioned at the lowermost end may be provided at arbitrary positions within the range R.

### (Method of manufacturing cell culture container)

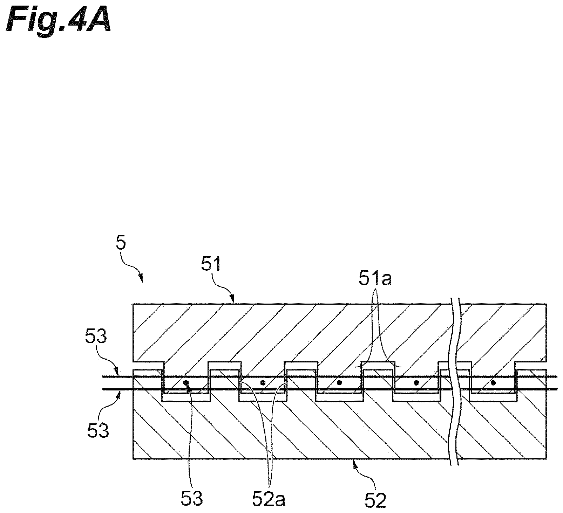

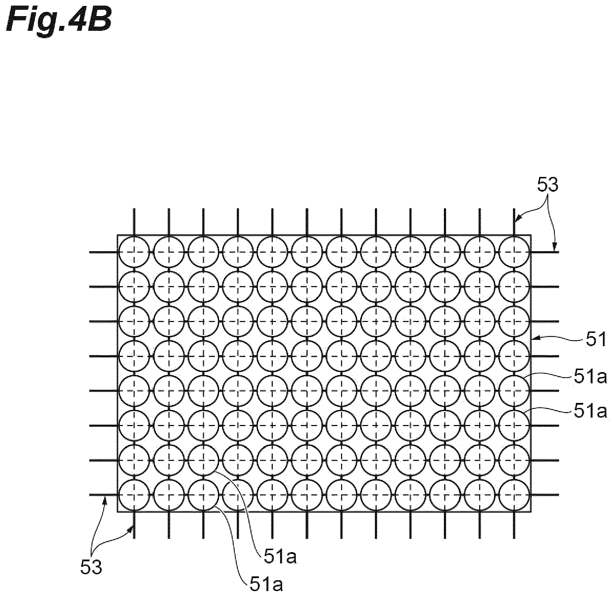

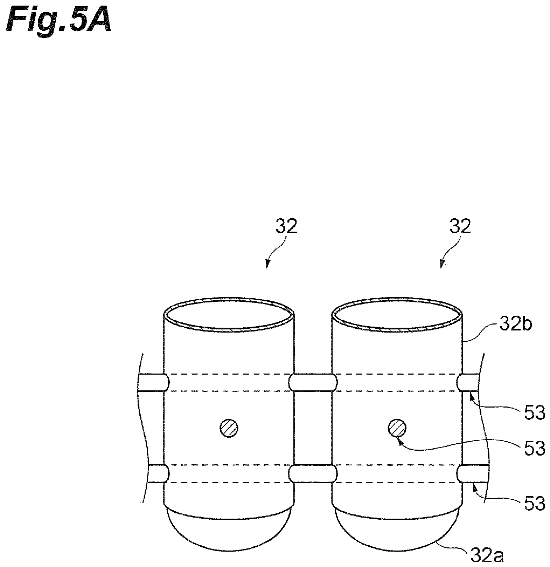

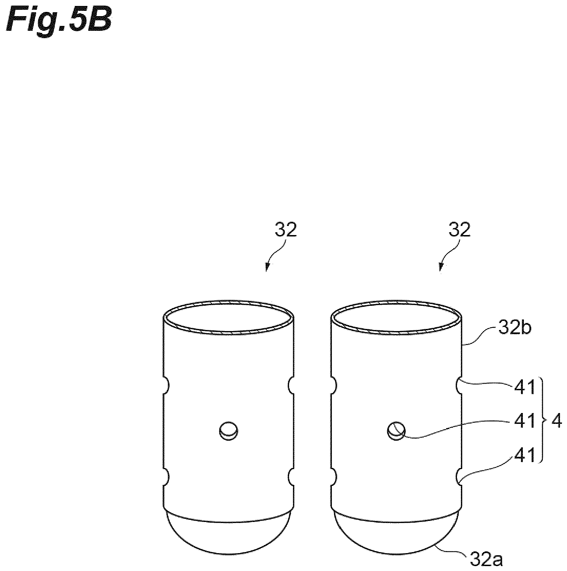

An example of a method of manufacturing the cell culture container 1 described above will be described. First, the culture solution container 2 is manufactured by molding (process of manufacturing a first container). For example, a pellet-like resin material is melted, and the melted resin material is poured into a mold and cured in that state. Thereby, the culture solution container 2 is manufactured. Next, the cell container 3 is manufactured by molding (process of manufacturing a second container). FIG. 4A is a cross-sectional view showing a mold 5 used in the method of manufacturing the cell culture container 1. In the process of manufacturing the second container, first, the mold 5 for molding is prepared. FIG. 4B is a view of an upper mold 51 and a core mold 53 shown in FIG. 4A when viewed from below. FIG. 5A and FIG. 5B are diagrams illustrating processes of the method of manufacturing the cell culture container 1.

As shown in FIG. 4A and FIG. 4B, the mold 5 includes the upper mold 51, a lower mold 52, and the plurality of core molds 53. The upper mold 51 and the lower mold 52 are molds for forming the outer shapes of the plate 31 and the well 32. Specifically, the upper mold 51 has a plurality of projection parts 51a having shapes corresponding to the outer shapes of the plurality of wells 32. The lower mold 52 has a plurality of recess parts 52a having shapes corresponding to the outer shapes of the plurality of wells 32. A through-hole through which the plurality of core molds 53 penetrate is formed in each of the projection parts 51a and the recess parts 52a.

The plurality of core molds 53 are molds for forming the liquid passage portion 4. FIG. 5A shows some wells 32 during molding, and FIG. 5B shows some wells 32 after molding. Each core mold 53 forms the hole 41. The core mold 53 is a rod-like member having an outer circumferential surface having the same shape as the hole 41. The core mold 53 is used when it penetrates the upper mold 51 and the lower mold 52. The plurality of core molds 53 may be arranged in parallel or in a state where the extending directions thereof may intersect, for example, depending on the position at which the hole 41 is formed in the well 32. The plurality of core molds 53 are arranged in a number corresponding to the number of holes 41 formed in each well 32. The hole 41 may be formed by one core mold 53 per well 32 or the holes 41 may be formed by a plurality of core molds 53 per well 32.

When the cell container 3 is manufactured, a pellet-like resin material is melted, and the melted resin material is poured into the mold 5, and cured in that state. Thereby, the cell container 3 in which the liquid passage portion 4 is provided in the well 32 is manufactured. As described above, the cell culture container 1 is completely manufactured. The cell container 3 may be manufactured before the culture solution container 2 is manufactured, and the culture solution container 2 and the cell container 3 may be manufactured in parallel.

### (Cell culture method)

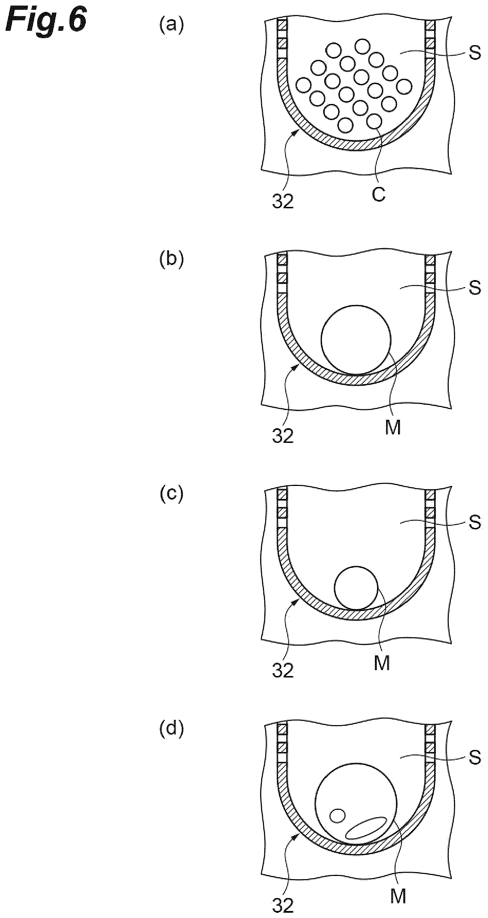

Next, as a cell culture method, a method of culturing cells C and cell masses M will be described. In this specification, "culture of cells C and cell masses M" means immersing of cells C in a culture solution S and aggregating, changing cell masses M that are immersed in a culture solution S, and differentiating them. FIG. 6 (a) portion to FIG. 6 (d) portion are schematic cross-sectional views showing changes in the culture state of cells C and cell masses M. Cells C immersed in the culture solution S (refer to (a) portion of FIG. 6) aggregate over several hours to several days to form cell masses M (refer to (b) portion of FIG. 6). When culture of the cell masses M additionally continues, aggregation is strengthened (refer to (c) portion of FIG. 6), and differentiation occurs (refer to (d) portion FIG. 6). This period varies depending on the type of cells C and details of differentiation, and may be several days to several weeks. During this period, cell masses M are appropriately optically observed. Hereinafter, details will be described.

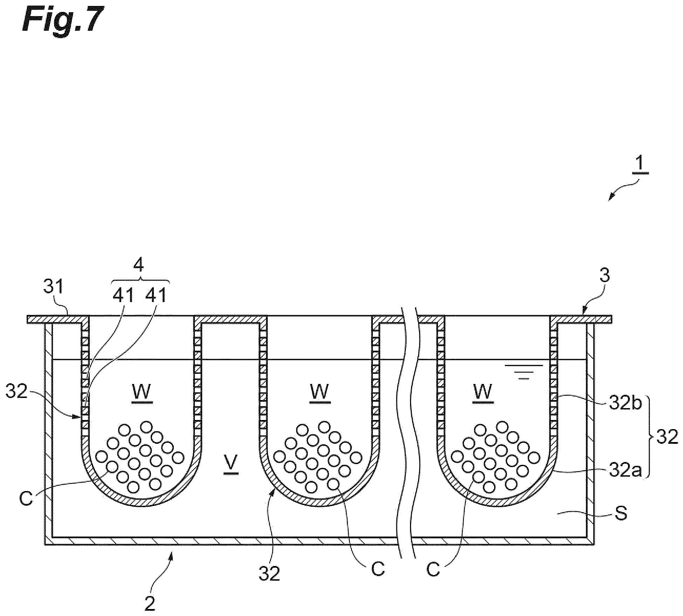

FIG. 7 and FIG. 8 are cross-sectional views schematically illustrating a culture process of a cell culture method according to one embodiment. First, the cell culture container 1 is prepared (preparation process). For example, the culture solution container 2 in which the culture solution S is accommodated, and the cell container 3 before the plurality of wells 32 are inserted into the accommodating space V of the culture solution container 2 are prepared. Next, as shown in FIG. 7 and FIG. 8, cells C are immersed in the culture solution S, and the cells C and cell masses M obtained by aggregating cells C are cultured (culturing process). In this process, when the plurality of wells 32 are inserted into the accommodating space V of the culture solution container 2 in which the culture solution S is accommodated, the culture solution S also enters the culture space W of the well 32 through the liquid passage portion 4. Thereby, the culture space W is filled with the culture solution S. Culture is started when cells C are seeded in the culture space W. In this case, the cells C are retained in the well 32. In that state, waiting is performed until a predetermined time has elapsed. The predetermined time is, for example, a time until cell masses M are obtained by aggregating cells C. The predetermined time is appropriately set according to the cells C to be cultured, and is, for example, several hours to several days.

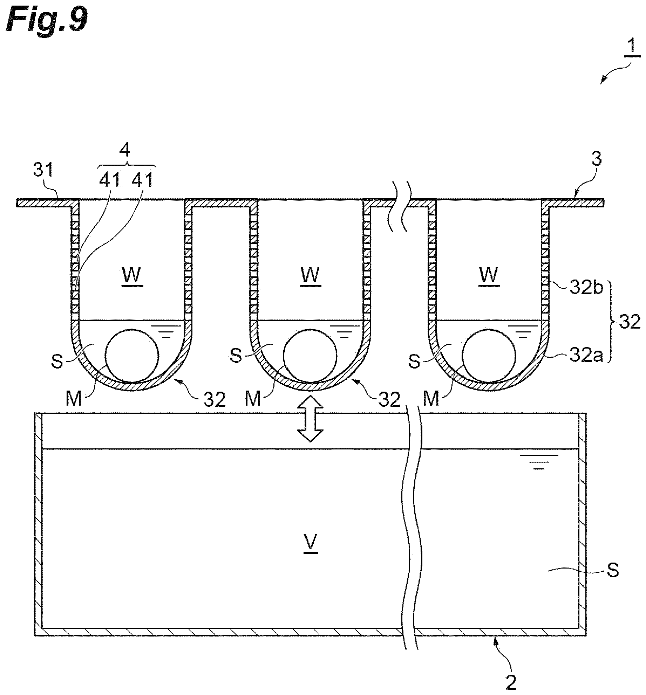

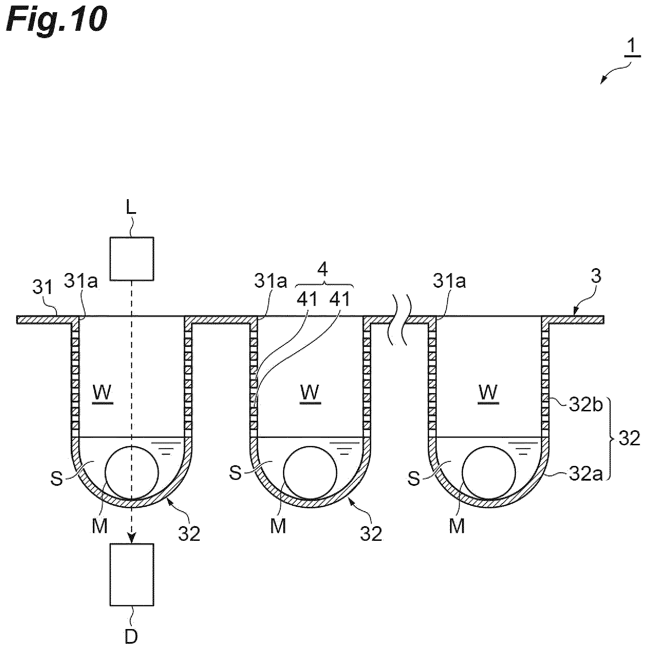

After the predetermined time has elapsed, as shown in FIG. 8, cell masses M are obtained. In this case, the cell masses M are retained in the well 32. Next, the cell masses M are observed (observation process). FIG. 9 and FIG. 10 are cross-sectional views schematically illustrating a culture process of the cell culture method according to one embodiment. In this process, first, as shown in FIG. 9, some of the culture solution S is discharged from the culture space W of the well 32. Specifically, the cell container 3 is removed from the culture solution container 2. According to this operation, some of the culture solution S is discharged from the well 32 through the liquid passage portion 4. Some of the culture solution S discharged here is some of the culture solution S present above the lower end (the hole 41 of the lowermost end) of the liquid passage portion 4 in the culture space W of the well 32. According to this operation, the parts of the culture solution S in the plurality of wells 32 are discharged at one time. According to this operation, the culture solution S remains only in the part including the bottom portion 32a of the well 32 (the part of the culture space W below the liquid passage portion 4). Thus, the state in which cell masses M are immersed in the culture solution S is maintained.

Next, as shown in FIG. 10, light is transmitted to cell masses M and observation is performed. For example, when the light source L emits light to cell masses M from above the well 32 (the connection hole 31a), the cell masses M are observed from below the well 32 (the bottom portion 32a) with an observation device D (e.g. a microscope). This operation is performed for each one or plurality of wells 32. When observation of cell masses M for all the wells 32 is completed, one observation process is completed.

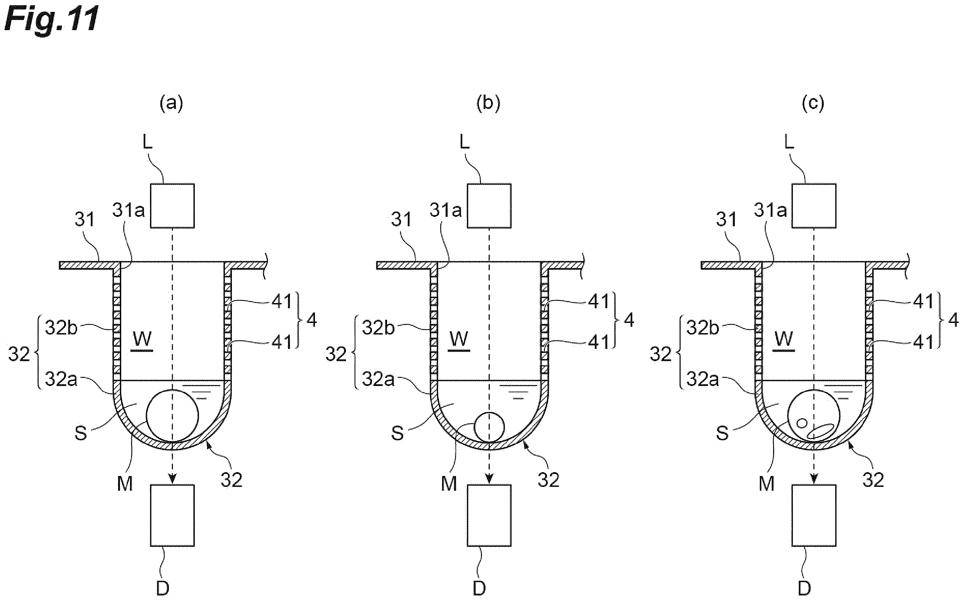

The above observation process may be performed only once or repeatedly performed at intervals. When the observation process is repeatedly performed, the state of change in the cell masses M may be appropriately (for example, periodically) observed. (a) portion of FIG. 11 to (c) portion of FIG. 11 are cross-sectional views schematically illustrating each observation process in which the state of change in cell masses M is observed. Examples of states of change in cell masses M include an initial state of cell masses M (refer to (a) portion of FIG. 11), a state in which aggregation of cell masses M is strengthened (refer to (b) portion of FIG. 11), a state in which aggregation of cell masses M is weakened (that is, a state in which aggregation of cell masses M is loosened), and a state in which cell masses M are differentiated (refer to (c) portion of FIG. 11). When the observation process is repeatedly performed, after each observation process is completed, the plurality of wells 32 are inserted again into the accommodating space V of the culture solution container 2 in which the culture solution S is accommodated, and thus the culture solution S enters the culture space W of the well 32 through the liquid passage portion 4. According to this operation, the culture spaces W of the plurality of wells 32 are filled with the culture solution S at one time. Thereby, the cell masses M are additionally cultured. Then, for example, after the cell masses M are formed, culture of the cell masses M continues, the observation process is performed again, and thus a state in which aggregation of cell masses M is strengthened as shown in (b) portion of FIG. 11, and a state in which cell masses M are differentiated as shown in (c) portion of FIG. 11 may be observed. When all the observation processes are completed, observation of the cell masses M is completed. Then, desired cell masses M are generated. The observation period varies depending on the type of cells and details of differentiation, and may be several days to several weeks.

### (Operations and effects)

Operations and effects of the cell culture container 1, the method of manufacturing the cell culture container 1, and the cell culture method described above will be described. In the cell culture container 1, the culture solution S is put in and taken out of the well 32 through the liquid passage portion 4 provided in the side wall 32b. Thus, the well 32 can be filled with the culture solution S simply by inserting the well 32 into the culture solution container 2 in which the culture solution S is accommodated. When the culture state of the cell masses M is observed, only some of the culture solution S in the well 32 can be easily discharged by simply ejecting the well 32 from the culture solution container 2 while the culture solution S is retained in the bottom portion 32a. Thus, in the procedure of culturing cells C and cell masses M, it is possible to efficiently observe the culture state of the cell masses M.

In the cell culture container 1 according to the present embodiment, the liquid passage portion 4 is the hole 41 that penetrates the side wall 32b. The hole 41 that penetrates the side wall 32b can realize a configuration that allows the culture solution S to pass through.

In the cell culture container 1 according to the present embodiment, the material forming the well 32 is polystyrene. Since polystyrene has a property of not easily adhering to cells C and cell masses M, it is suitable for culturing cells C and cell masses M. When the well 32 is formed of polystyrene, for example, the cultured cell masses and the like can be easily removed from the well 32.

In the cell culture container 1 according to the present embodiment, the cell container 3 has the plurality of wells 32 connected to each other, and among the plurality of wells 32, the height H3 from the lower end of the bottom portion 32a to the lower end of the liquid passage portion 4 is constant. For example, in a configuration having a plurality of wells 32, when the culture state of cell masses M is observed, if the culture solution S in the well 32 is manually removed with a syringe or the like, it is difficult to keep the amount of the culture solution S remaining in the well 32 constant. In addition, as the number of wells 32 increases, time and effort increase. On the other hand, the cell culture container 1 according to the present embodiment, according to the configuration in which the height H3 is constant among the plurality of wells 32, by simply ejecting the well 32 from the culture solution container 2, it is possible to easily discharge only some of the culture solution S in the well 32 while a certain amount of the culture solution S is retained in the bottom portion 32a of the plurality of wells 32.

In the cell culture container 1 according to the present embodiment, the liquid passage portion 4 may be provided in the side wall 32b such that the height from the lower end of the bottom portion 32a to the lower end of the liquid passage portion 4 is 1.0 mm or more and 5.0 mm or less. When the liquid passage portion 4 is positioned in such a range with respect to the bottom portion 32a that can retain the culture solution S, even if the culture solution S is discharged through the liquid passage portion 4 when cell masses and the like are observed, the cell masses and the like can be prevented from drying out, and an observation operation can be easily performed. More specifically, when the height to the lower end of the liquid passage portion 4 is 1.0 mm or more, even if cell masses and the like are observed, the cell masses and the like retained in the bottom portion 32a can be more reliably immersed in the culture solution. In addition, if the height to the lower end of the liquid passage portion 4 is 5.0 mm or less, when cell masses and the like are observed, it is possible to perform discharge more easily so that the amount of the culture solution that interferes with observation is more appropriate.

The cell culture method according to the present embodiment includes a process of preparing the cell culture container 1, a process of immersing cells C retained in the well 32 in the culture solution S accommodated in the culture solution container 2 and culturing cells C and cell masses M obtained by aggregating cells C, and a process of observing cell masses M. In the observation process, cell masses M in the well 32 are observed when some of the culture solution S is discharged from the well 32 through the liquid passage portion 4 by removing the cell container 3 from the culture solution container 2. Thereby, in the observation process, some of the culture solution S can be easily discharged from the well 32. Since cell masses M in the well 32 are observed when some of the culture solution S is discharged, it is possible to prevent the culture solution S from interfering with observation of cell masses M. Thus, in the procedure of culturing cells C and cell masses M, it is possible to efficiently observe cell masses M. When a process of additionally culturing cell masses M and performing observation is appropriately repeatedly performed after the cell masses M are obtained, in each observation process. Since some of the culture solution S can be easily discharged from the plurality of wells 32, each observation process can be efficiently performed. Thus, it is possible to efficiently observe a desired culture state of the cell masses M. As described above, the cell culture method according to the present embodiment is particularly advantage when observation of the culture state of the cell masses M is appropriately repeated.

A method of manufacturing the cell culture container 1 according to the present embodiment includes a process of manufacturing the cell container 3 by molding, and the process includes a process of preparing the mold 5 including the upper mold 51 having the projection part 51a having a shape corresponding to an outer shape of the well 32, the lower mold 52 that is arranged facing the projection part 51a and has the recess part 52a having a shape corresponding to an outer shape of the well 32, and the core mold 53 that penetrates the projection part 51a and the recess part 52a, and a process of curing a resin material poured into the mold 5 and molding the well 32, and the hole 41 may be formed as the liquid passage portion 4 with the core mold 53. According to this configuration, since the hole 41 as the liquid passage portion 4 is formed in the well 32 of the cell container 3 manufactured by molding, the cell culture container 1 can be easily formed. Thus, when the manufactured cell culture container 1 is used, in the procedure of culturing cells C and cell masses M, it is possible to efficiently observe the culture state of the cell masses M. If cell masses M are continuously cultured and the state of change in the cell masses M is appropriately observed, when the manufactured cell culture container 1 is used, it is possible to efficiently observe a desired culture state of the cell masses M.

### (Modification examples)

The above embodiments describe one embodiment of a cell culture container, a method of manufacturing a cell culture container, and a method of culturing cell masses according to the present disclosure. The cell culture container, the method of manufacturing a cell culture container, and the cell culture method according to the present disclosure may be obtained by arbitrarily modifying the above embodiments.

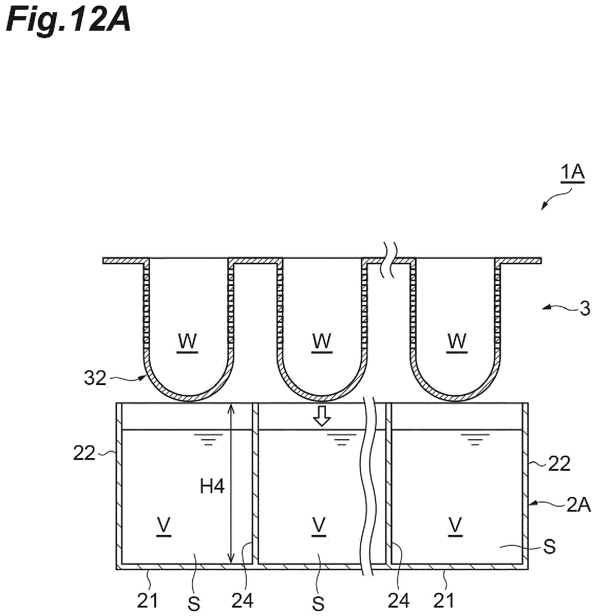

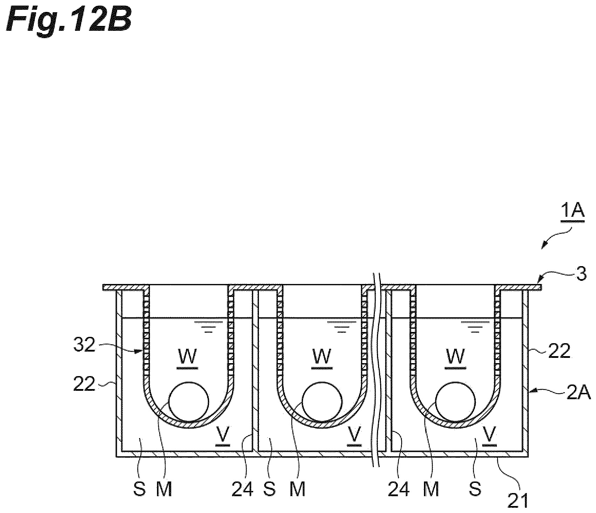

For example, in the above embodiment, the culture solution container 2 has one continuous accommodating space V, but may have a plurality of accommodating spaces V. FIG. 12A and FIG. 12B are cross-sectional views schematically showing a cell culture container 1A according to a modification example. The cell culture container 1A has the same configuration as the cell culture container 1 except that it has a culture solution container 2A in place of the culture solution container 2.

The culture solution container 2A has a plurality of accommodating spaces V. Specifically, the culture solution container 2A further includes a plurality of partition walls 24 arranged in the space defined by the bottom plate 21 and the four side walls 22. The partition walls 24 water-tightly partition the adjacent accommodating spaces V. In other words, the plurality of accommodating spaces V are separated from each other by the plurality of partition walls 24. Some of the plurality of wells 32 can be put in and taken out of the accommodating spaces V. As an example, the culture solution container 2A has twelve accommodating spaces V, and the size of one accommodating space V is a size at which one column (eight out of ninety six) of the wells 32 can be accommodated.

The partition wall 24 stands from the top surface of the bottom plate 21. Each partition wall 24 is provided at a position corresponding to each accommodating space V on the top surface of the bottom plate 21. The thickness of the partition wall 24 is, for example, 1 mm. A height H4 of the partition wall 24 is, for example, 14.5 mm. The material forming the partition wall 24 may be the same as the material forming the bottom plate 21. The number of accommodating spaces V may be set arbitrarily. The culture solution container 2A may have the same number (ninety six) of accommodating spaces V as the well 32, or may have only two accommodating spaces V. When the culture solution container 2A has only two accommodating spaces V, it may have only one partition wall 24.

Also in the cell culture container 1A, since the culture solution S is put in and taken out of the well 32 through the liquid passage portion 4 provided in the side wall 32b, the same operations and effects as those of the cell culture container 1 are obtained. According to the culture solution container 2A, since the plurality of accommodating spaces V are water-tightly partitioned, the culture solution S does not enter the wells 32 accommodated in the accommodating spaces V from the other accommodating spaces V. Thus, it is possible to avoid contamination caused by the culture solution S accommodated in the other accommodating spaces V.

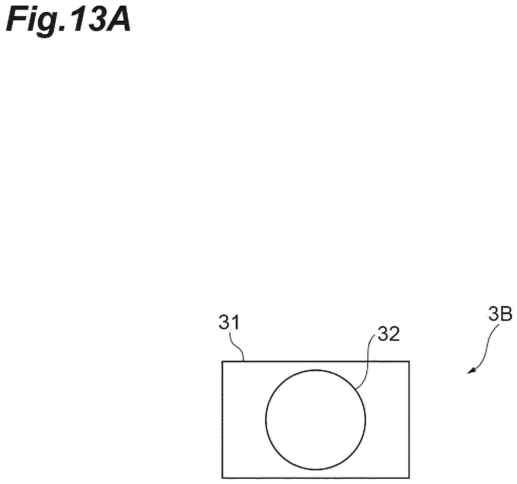

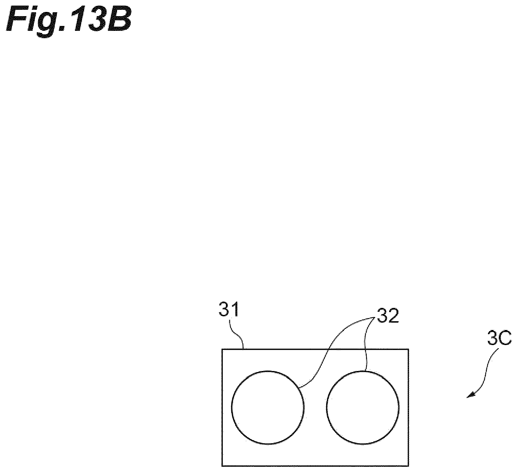

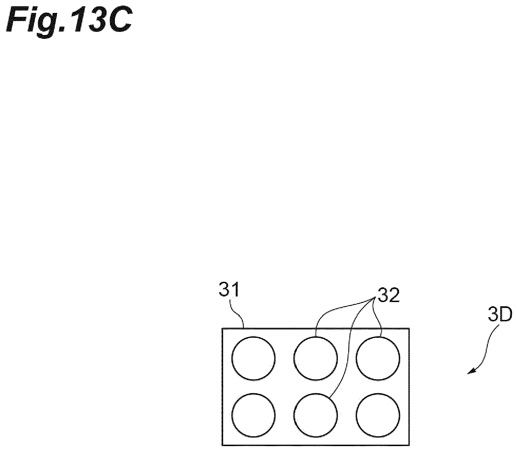

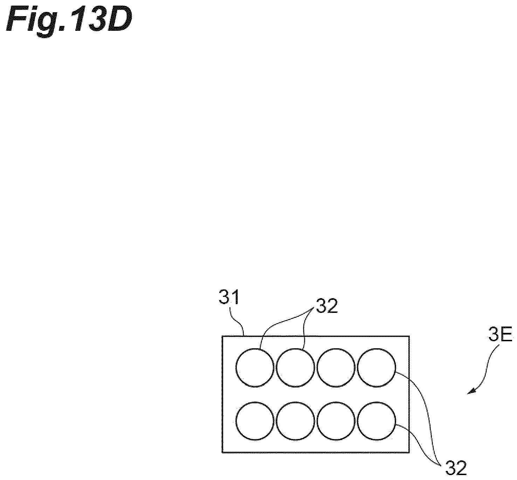

The number of wells 32 in the cell container 3 is an example, and may be appropriately set. FIG. 13A to FIG. 13D are plan views schematically showing the cell containers 3B to 3E according to a modification example. The cell containers 3B to 3E have the same configuration as the cell container 3 except that the number of wells 32 is different from that of the cell container 3. As shown in FIG. 13A, the cell culture container 1 may include a cell container 3B having only one well 32 in place of the cell container 3. As shown in FIG. 13B, the cell culture containers 1 and 1A may include a cell container 3C having two wells 32 in place of the cell container 3. As shown in FIG. 13C, the cell culture containers 1 and 1A may include the cell container 3C having six wells 32 in place of the cell container 3. As shown in FIG. 13D, the cell culture containers 1 and 1A may include the cell container 3C having eight wells 32 in place of the cell container 3. The cell container in the cell culture containers 1 and 1A may have a number of wells 32 other than the above number, and may have, for example, three hundred eighty four wells 32.

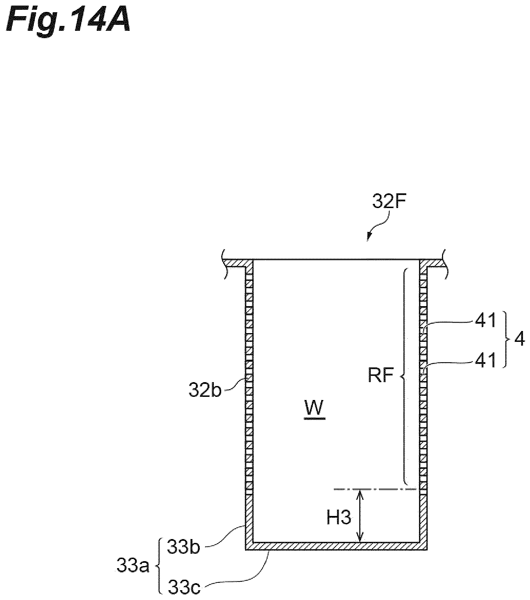

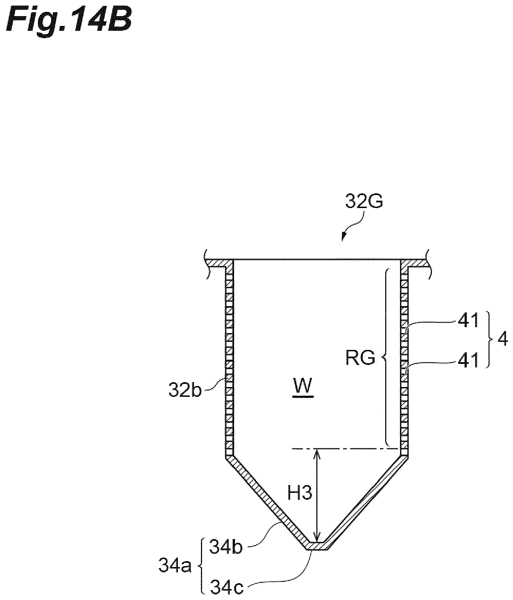

The shape of the bottom portion 32a of the well 32 is an example, and may be appropriately changed. FIG. 14A and FIG. 14B are cross-sectional views schematically showing wells 32F and 32G according to a modification example. The well 32F shown in FIG. 14A has the same configuration as the well 32 except that it has a bottom portion 33a in place of the bottom portion 32a. The bottom portion 33a has a circumferential wall 33b and a circular bottom wall 33c that blocks below the circumferential wall 33b. The circumferential wall 33b has a cylindrical shape having the same diameter as the inner diameter of the side wall 32b. In this case, the overall shape of the culture space W defined by the bottom portion 33a and the side wall 32b is a columnar shape. The liquid passage portion 4 (the hole 41) is provided in a range RF separated from the tip (lower end) of the bottom portion 33a by the predetermined height H3.

The well 32G shown in FIG. 14B has the same configuration as the well 32 except that it has a bottom portion 34a in place of the bottom portion 32a. The bottom portion 34a has a circumferential wall 34b and a circular bottom portion wall 34c that blocks below the circumferential wall 34b. The circumferential wall 34b has a shape tapered in a conical shape from the lower end of the side wall 32b. The liquid passage portion 4 (the hole 41) is provided in a range RG separated from the tip (lower end) of the bottom portion 34a by the predetermined height H3.

In the wells 32F and 32G, the culture solution S is put in and taken out of the wells 32F and 32G through the liquid passage portion 4 provided in the side wall 32b. Thus, in the procedure of culturing cells C and cell masses M, it is possible to efficiently observe the culture state of the cell masses M. When cell masses M are continuously cultured and the state of change in the cell masses M is appropriately observed, it is possible to efficiently observe a desired culture state of the cell masses M.

The configuration of the liquid passage portion 4 is an example, and may be appropriately changed. The shape of the hole 41 of the liquid passage portion 4 may be appropriately changed. The shape of the hole 41 may be, for example, an elliptical shape or a semicircular shape. Alternatively, the liquid passage portion 4 may be a combination of the plurality of holes 41 having different shapes.

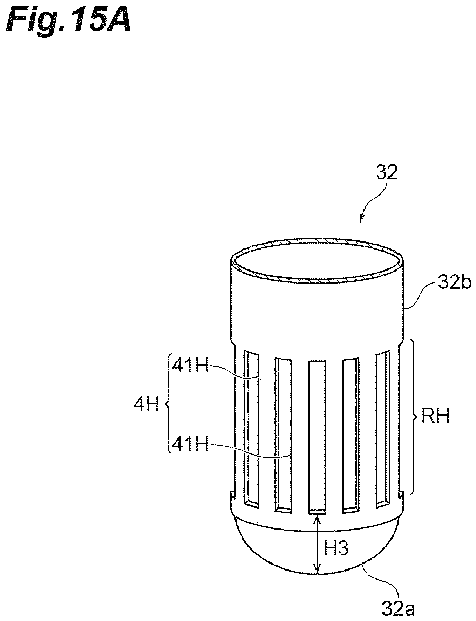

FIG. 15A to FIG. 15C are perspective views schematically showing the liquid passage portions 4H, 4J, and 4K according to a modification example. The liquid passage portion 4H shown in FIG. 15A is provided in a region (a range RH) of a part of the side wall 32b. The lower end of the range RH is a position separated from the tip (lower end) of the bottom portion 32a by the height H3. The height of the range RH is, for example, 7.0 mm. The liquid passage portion 4H is one or a plurality of long holes 41H (slits). The long hole 41H penetrates the side wall 32b. The long hole 41H extends in the extending direction (vertical direction) of the side wall 32b. The shape of the long hole 41H is, for example, a rectangular shape. The height (in this modification example, the size in the longitudinal direction) of the long hole 41H is 0.01 mm or more and 7.0 mm or less, and as an example, the same as the range RH (7.0 mm). The width (in this modification example, the size in the lateral direction) of the long hole 41H is, for example, 0.01 mm or more and 6.0 mm or less. Alternatively, the width of the long hole 41H may be 3.0 mm or less, or 1.0 mm or less.

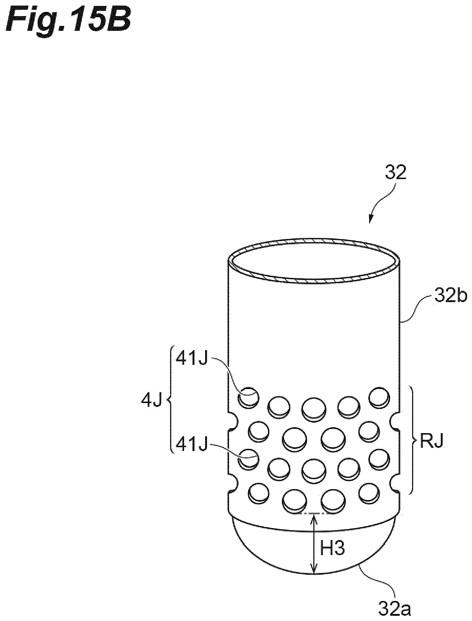

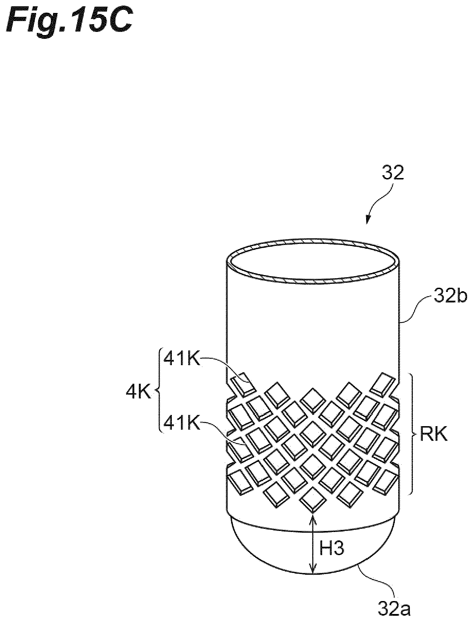

The liquid passage portion 4J shown in FIG. 15B is provided in a region (a range RJ) of a part of the side wall 32b. The range RJ is the same as, for example, the range RH. The liquid passage portions 4J are the plurality of holes 41 densely provided in the range RJ. The liquid passage portion 4K shown in FIG. 15C is provided in a region (a range RK) of a part of the side wall 32b. The range RK is the same as, for example, the ranges RH and RJ. The liquid passage portions 4K are a plurality of holes 41K densely provided in the range RK. The hole 41K has the same configuration as the hole 41 except that it has a rhombus shape when viewed in the penetration direction. The height of the hole 41K is, for example, 0.01 mm or more and 7.0 mm or less. The width of the hole 41K is, for example, 0.01 mm or more and 6.0 mm or less. Alternatively, the width of the hole 41K may be 3.0 mm or less or 1.0 mm or less.

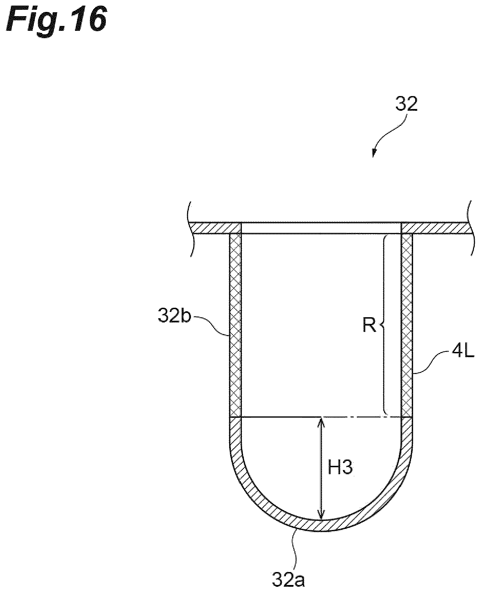

The liquid passage portion is not limited to the hole. FIG. 16 is a cross-sectional view schematically showing a liquid passage portion 4L according to a modification example. As shown in FIG. 16, the liquid passage portion 4L may be formed by forming at least a part of a region (the range R) of the side wall 32b with a material different from the bottom portion 32a. In the example shown in FIG. 16, the range R is the entire region of the side wall 32b. In this case, the material forming the liquid passage portion 4L is, for example, a porous polymer material through which the culture solution S passes. Thereby, the region of the side wall 32b formed of a porous polymer material can realize a configuration that allows the culture solution S to pass through. Thus, in this configuration, since the culture solution S is put in and taken out of the well 32 through the liquid passage portion 4L, in the procedure of culturing cells C, it is possible to efficiently observe the culture state of cell masses M. Similarly, in this configuration, when cell masses M are continuously cultured and the state of change in the cell masses M is appropriately observed, it is possible to efficiently observe a desired culture state of the cell masses M.

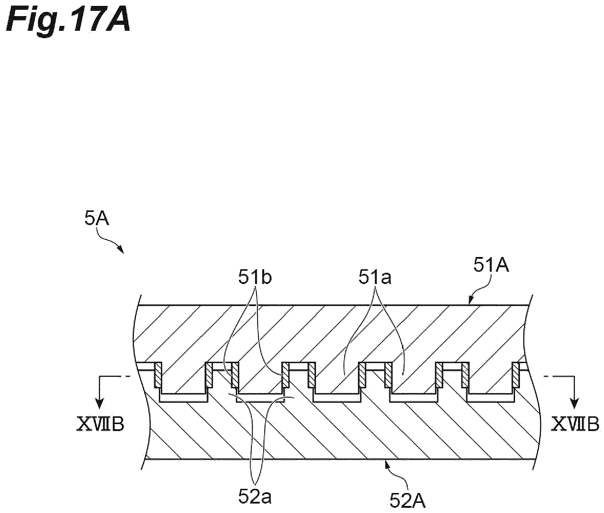

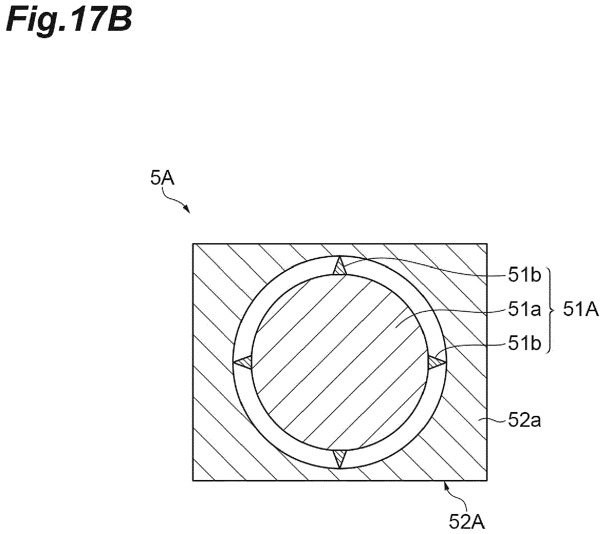

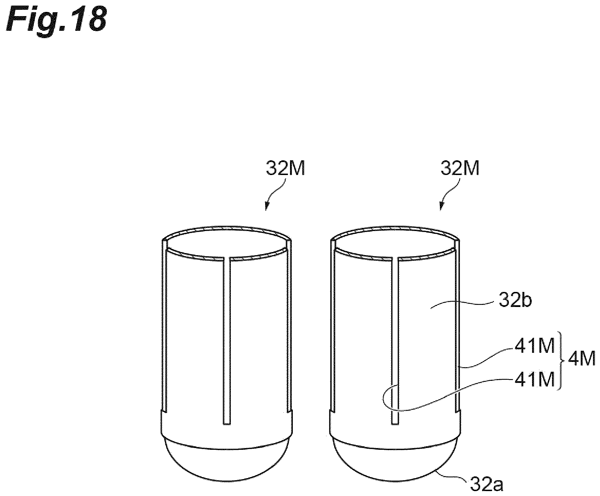

The method of manufacturing a cell culture container according to the above embodiment is an example, and may be appropriately changed. A second container manufacturing process may be performed using the following mold 5Ain place of the mold 5. FIG. 17Ais a cross-sectional view showing the mold 5A used in a method of manufacturing a cell culture container according to a modification example. FIG. 17B is a cross-sectional view along the line XVIIB-XVIIB in FIG. 17A. FIG. 18 is a diagram showing some wells 32M after molding by the method of manufacturing a cell culture container according to the modification example.

As shown in FIG. 17A and FIG. 17B, the mold 5A includes an upper mold 51A and a lower mold 52A. Like the upper mold 51, the upper mold 51A has a plurality of wells 32 and a plurality of projection parts 51a having the same shape. Like the lower mold 52, the lower mold 52A has a plurality of wells 32 and a plurality of recess parts 52a having the same shape. The upper mold 51A has one or a plurality of (four in the example in FIG. 17B) of protrusions 51b that further protrude from the surface of the projection part 51a. The protrusion 51b extends in the vertical direction (that is, the direction in which the upper mold 51A and the lower mold 52A face each other) over the range in which the liquid passage portion (a liquid passage portion 4M to be described below) is provided.

As shown in FIG. 18, the well 32M has the same configuration as the well 32 except that it has the liquid passage portion 4M in place of the liquid passage portion 4. The protrusion 51b forms a slit 41M as the liquid passage portion 4M. One protrusion 51b may form one slit 41M per well 32M, or a plurality of protrusions 51b may form a plurality of slits 41M per well 32. When the cell container 3 is manufactured, a pellet-like resin material is melted, and the melted resin material is poured into the mold 5A and cured in that state. Thereby, the cell container 3 in which the liquid passage portion 4M is provided in the well 32M is manufactured.

According to this configuration, since the slit 41M as the liquid passage portion 4M is formed in the well 32M of the cell container 3 manufactured by molding, the cell culture container 1 can be easily formed. Thus, when the manufactured cell culture container 1 is used, in the procedure of culturing cells C and cell masses M, it is possible to efficiently observe the cell masses M. When cell masses M are continuously cultured and the state of change in the cell masses M is appropriately observed, it is possible to efficiently observe a desired culture state of the cell masses M.

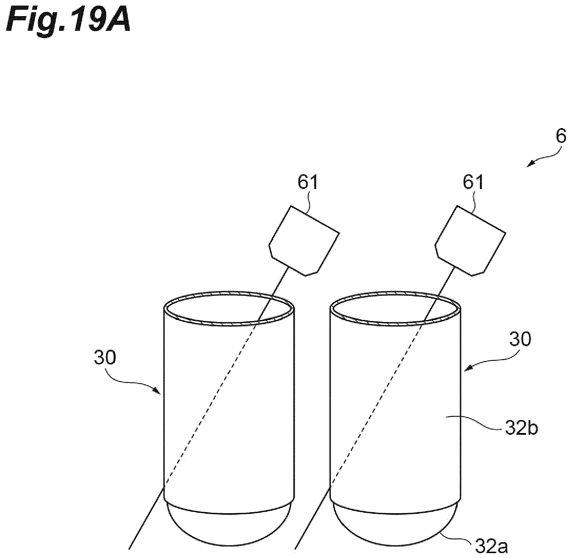

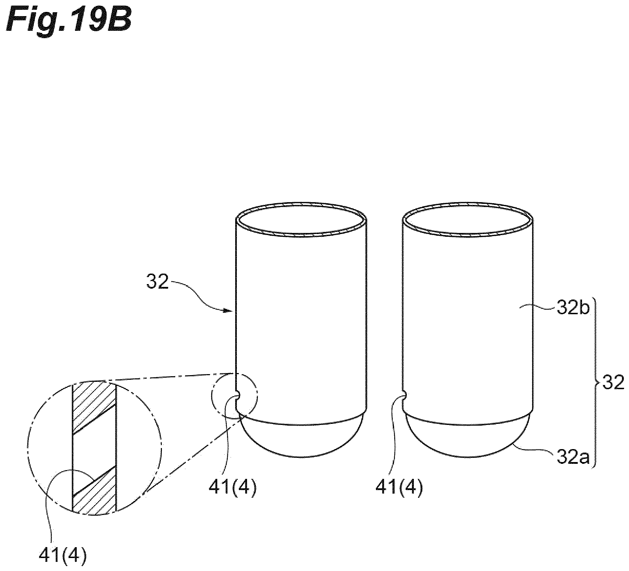

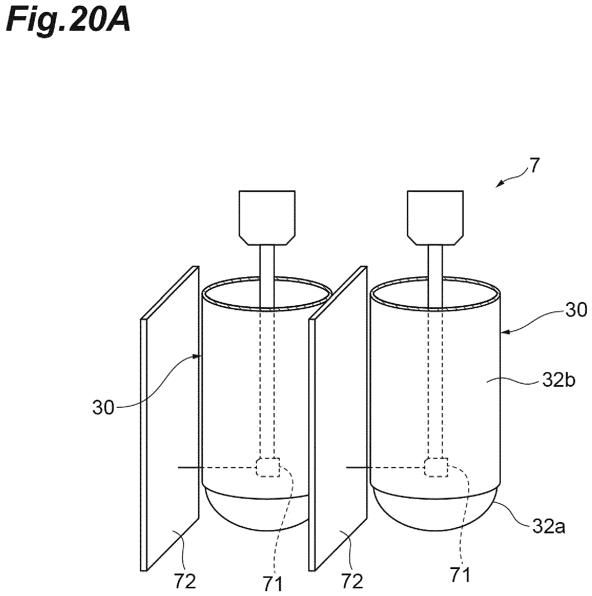

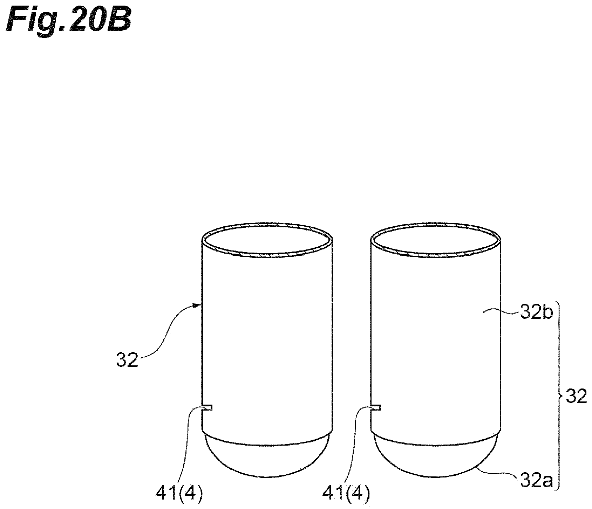

The liquid passage portion 4 may be formed by laser emission. FIG. 19A and FIG. 19B, and FIG. 20A and FIG. 20B are diagrams illustrating processes of a method of manufacturing a cell culture container according to a modification example. FIG. 19A shows some wells 32 in a process of emitting a laser beam, and FIG. 19B shows some wells 32 after molding according to the process shown in FIG. 19A. When the cell container 3 is manufactured, first, a molded product 30 is molded by molding (a process of molding a mold). The molded product 30 exhibits the outer shape of the plate 31 and the plurality of wells 32. Next, the hole 41 is formed in the molded product 30 by laser emission (a process of emitting a laser beam).

As shown in FIG. 19A, in the process of emitting a laser beam, a laser device 6 is used. The laser device 6 has a plurality of laser heads 61 arranged corresponding to the plurality of wells 32. Each laser head 61 outputs a laser beam in a direction diagonally intersecting the side wall 32b. Thereby, as shown in an enlarged manner in FIG. 19B, the hole 41 that diagonally penetrates the side wall 32b may be formed.

Alternatively, a process of emitting a laser beam may be performed as follows. FIG. 20A shows some wells 32 in a process of emitting another laser beam, and FIG. 20B shows some wells 32 after molding in FIG. 20A. As shown in FIG. 20A, in the process of emitting a laser beam, a laser device 7 may be used in place of the laser device 6. The laser device 7 has a plurality of laser heads 71 arranged corresponding to the plurality of wells 32. Each laser head 71 outputs a laser beam in a direction orthogonal to the side wall 32b. A shielding plate 72 may be arranged between two adjacent molded products 30. Alternatively, the shielding plate 72 is not arranged and thus a laser beam may be emitted over the plurality of molded products 30. Thereby, as shown in FIG. 20B, the hole 41 that penetrates the side wall 32b in a thickness direction of the side wall 32b may be formed.

When formation of all the holes 41 as the liquid passage portions 4 is completed according to any of the processes of emitting a laser beam, the cell container 3 in which the liquid passage portion 4 is provided in the well 32 is manufactured. According to these configurations, since the hole 41 as the liquid passage portion 4 is formed in the manufactured well 32 by laser emission, the cell culture container 1 can be easily formed. Thus, when the manufactured cell culture container 1 is used, in the procedure of culturing cells C and cell masses M, it is possible to efficiently observe the cell masses M. When cell masses M are continuously cultured and the state of change in the cell masses M is appropriately observed, it is possible to efficiently observe a desired culture state of the cell masses M.

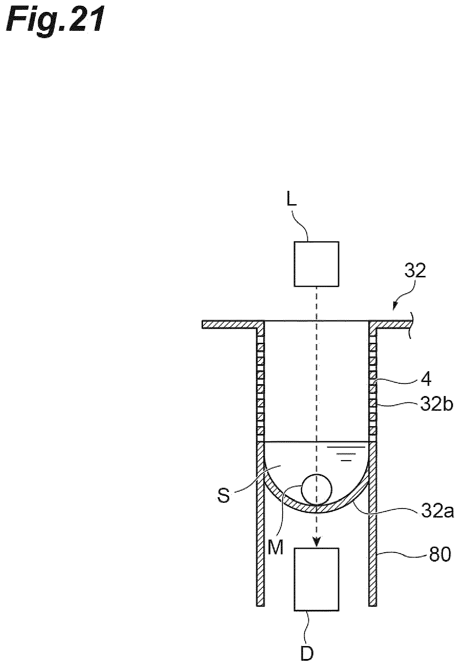

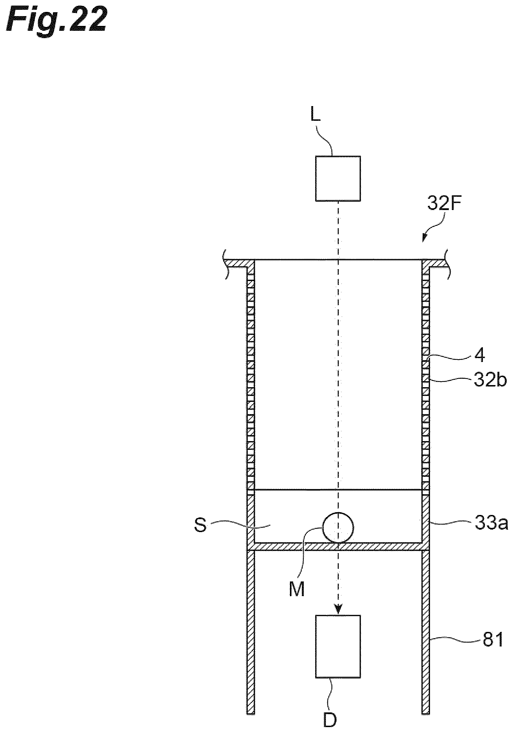

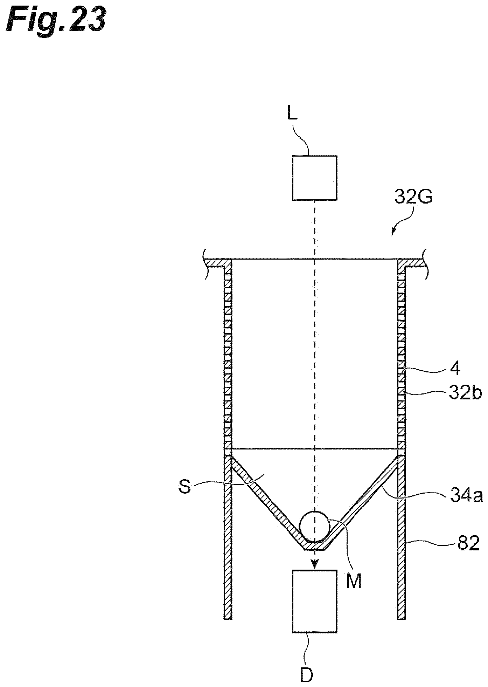

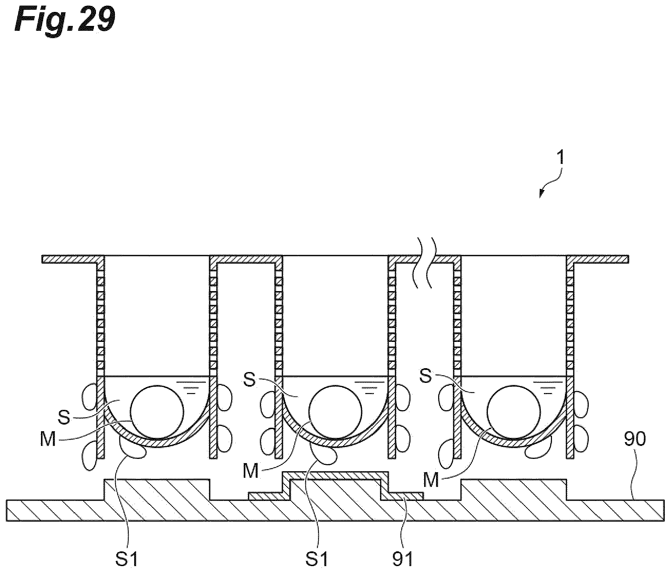

FIG. 21 to FIG. 28 are diagrams schematically showing a well according to another modification example. The well 32 is removed from the culture solution container 2 when a cell mass M is observed with light emitted from a light source L. In this case, it is considered that the culture solution S discharged from the liquid passage portion 4 drips (refer to FIG. 29), and adheres to the outside of the bottom portion 32a of the well 32. In this case, droplets of the culture solution S are located on the line connecting the light source L, the cell mass M, and the observation device D, and may interfere with observation of the cell mass M. Thus, for example, as shown in FIG. 21, FIG. 22 and FIG. 23, guide members 80, 81, and 82 that guide a flow of the culture solution S discharged from the liquid passage portion 4 may be provided on the outer circumferential surface of the bottom portions 32a, 33a, and 34a, respectively. The guide members 80 to 82 are, for example, cylindrical members extending in a circumferential shape. Each of the guide members 80 to 82 is provided on the outer circumferential surface of the wells 32, 32F, and 32G in a region closer to the bottom portions 32a, 33a, and 34a than the liquid passage portion 4, and extends to the side (that is, the lower side in the drawing) opposite to the liquid passage portion 4. Each of the guide members 80 to 82 may have a length at which the culture solution S does not transmit to the bottom portions 32a, 33a, and 34a, or may have a length equal to or longer than that of the lower end of the bottom portions 32a, 33a, and 34a. The length of the guide members 80 to 82 may be, for example, 0.5 mm or more and 10 mm or less. Thereby, it is possible to prevent interference with observation of the cell mass M due to dripping of the culture solution S discharged from the liquid passage portion 4 when the cell container 3 having the well 32, 32F or 32G is removed from the culture solution container 2.

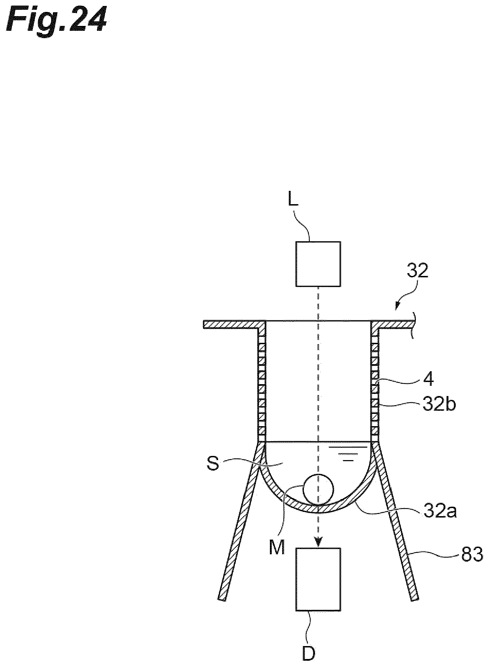

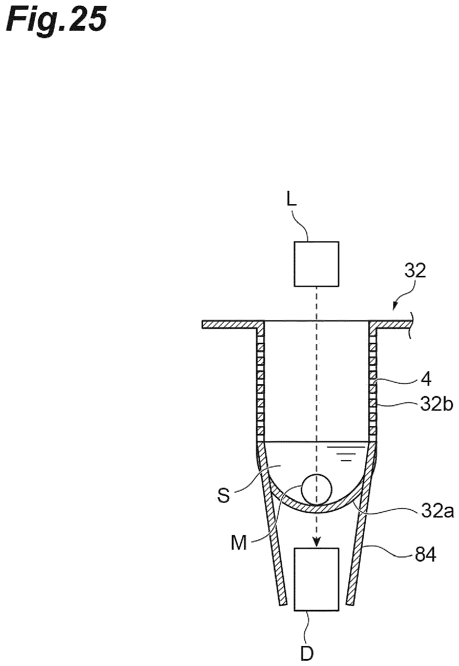

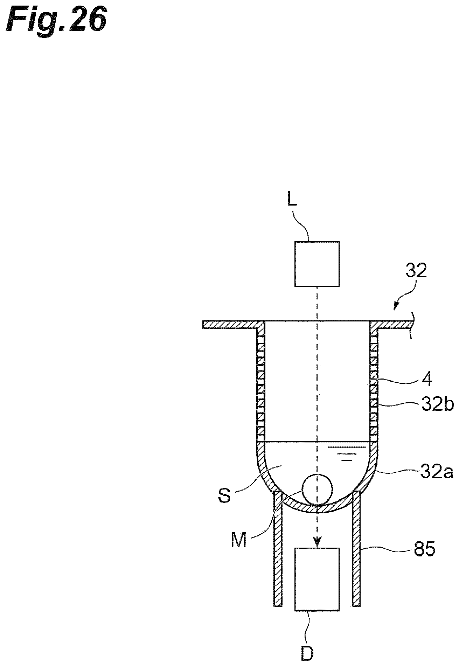

As shown in FIG. 24, a guide member 83 may be provided so that it is inclined outward from the well 32. In this case, the guide member 83 may be inclined outward at an inclination angle of 90 degrees or less with respect to the vertical direction. On the other hand, as shown in FIG. 25, a guide member 84 may be provided so that it is inclined inward from the well 32. In this case, it may be inclined inward at an inclination angle of 45 degrees or less with respect to the vertical direction. At this angle, installation of the observation device D is not interfered with. As shown in FIG. 21 and the like, the guide member 80 may extend linearly downward from the side wall 32b, and as shown in FIG. 26, a guide member 85 may be positioned further inside than the side wall 32b in the radial direction and extend downward from the intermediate part of the bottom portion 32a. However, in the configuration shown in FIG. 26, the distance between facing portions of the guide member 85 may be larger than the diameter of the cell mass M to be observed. For example, when the diameter of the cell mass M is 1 mm or less, the location at which the guide member 85 is installed may be a region within a range of 1 mm or more in diameter from the center of the bottom portion 32a. Thereby, it is possible to prevent interference with observation of the cell mass M due to the observation device D

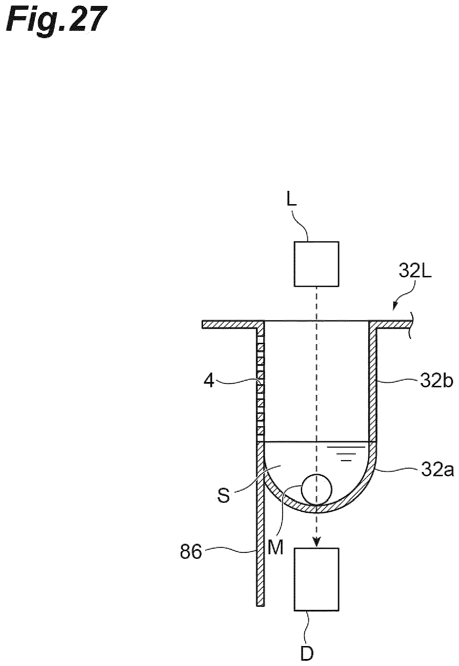

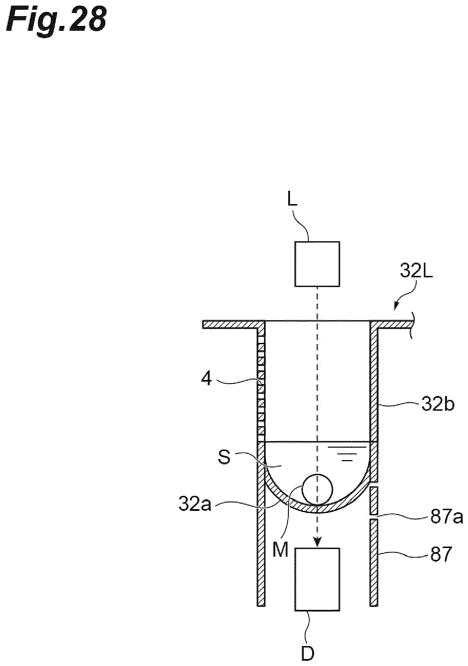

Since the guide member is configured to prevent liquid dripping, it need not be arranged on the entire circumference of the bottom portion 32a. For example, as shown in FIG. 27, in the case of a well 32L in which the liquid passage portion 4 is provided on one of the side walls 32b, a guide member 86 may be provided only on the side in which the liquid passage portion 4 is provided. In this case, the guide member 86 may be a member having a semi-cylindrical shape or an arc-shaped cross section. Even if the guide member is provided to extend in the circumferential direction, as shown in FIG. 28, in the case of the well 32L in which the liquid passage portion 4 is provided on one of the side walls 32b, a hole 87a may be provided in a part of a guide member 87 on the side in which the liquid passage portion 4 is not provided.

In addition to or in place of provision of the guide member 80 and the like, as shown in FIG. 29, the cell culture container 1 may further include a liquid dripping removal device 90. The liquid dripping removal device 90 is formed of, for example, a resin or a metal. For example, a gauze 91 for adsorbing droplets S1 of the culture solution S may be installed on the liquid dripping removal device 90. The base itself of the liquid dripping removal device 90 may be formed of an absorbent material (sponge material), and droplets S1 of the culture solution S adhered to the outer circumference of the bottom portion 32a of the well 32 of the cell culture container 1 may be absorbed and removed before observation. The surface of the liquid dripping removal device 90 may have an uneven shape in order to avoid the guide member 80 and the like, or may be flat when the guide member 80 or the like is not provided. The gauze 91 arranged on or above the base of the liquid dripping removal device 90 may be formed of a material that can be sterilized in order to prevent contamination.

The above embodiments and the modification examples may be combined with each other.

### Reference Signs List

1, 1A Cell culture container
2, 2A Culture solution container (first container)
3, 3B, 3C, 3D, 3E Cell container (second container)
4, 4H, 4J, 4K, 4L, 4M Liquid passage portion
5, 5A Mold
6, 7 Laser device
21 Bottom plate
22 Side wall
23 Opening
24 Partition wall
30 Molded product
31 Plate
31a Connection hole
32, 32F, 32G, 32M, 32L Well
32a, 33a, 34a Bottom portion
32b Side wall
33b, 34b Circumferential wall
33c, 34c Bottom wall
41, 41K Hole
41H Long hole
41M Slit
51, 51A Upper mold
51a Projection part
51b Protrusion
52, 52A Lower mold
52a Recess part
53 Core mold
61, 71 Laser head
72 Shielding plate
80, 81, 82, 83, 84, 85, 86, 87 Guide member
87a Hole
90 Liquid dripping removal device
91 Gauze
C Cell
D Observation device
H1, H2, H3, H4 Height
L Light source
M Cell mass
P Pitch
R, RF, RG, RH, RJ, RK Range
S Culture solution
S1 Droplets
V Accommodating space
W Culture space
ϕ1, cp2 Inner diameter

## Claims

1. A cell culture container, comprising:
a first container configured to accommodate a culture solution; and
a second container including one or more wells that are able to be put in and taken out of the first container while retaining one or both of cells and cell masses cultured in the culture solution,
wherein one or more wells each include
a bottom portion that is able to retain the culture solution, and
a side wall that extends from a peripheral portion of the bottom portion, and
wherein a liquid passage portion through which the culture solution accommodated in the first container is able to pass is provided in the side wall.

2. The cell culture container according to claim 1,
wherein the liquid passage portion is one or more holes that penetrate the side wall.

3. The cell culture container according to claim 1 or 2,
wherein the material forming the one or more wells is polystyrene.

4. The cell culture container according to claim 1,
wherein the liquid passage portion is at least a part of a region of the side wall, and
wherein the region is formed of a porous polymer material that allows the culture solution to pass through.

5. The cell culture container according to any one of claims 1 to 4,
wherein the one or more wells are a plurality of wells connected to each other.

6. The cell culture container according to claim 5,
wherein the heights from lower ends of the bottom portions of the plurality of wells to lower ends of the liquid passage portions are constant.

7. The cell culture container according to claim 5 or 6,
wherein the first container includes
a plurality of accommodating spaces that are able to be put in and taken out of some of the plurality of wells, and
one or more partition walls that separate the plurality of accommodating spaces from each other.

8. The cell culture container according to any one of claims 1 to 7,
wherein the height from a lower end of the bottom portion to a lower end of the liquid passage portion is 1.0 mm or more and 5.0 mm or less.

9. The cell culture container according to any one of claims 1 to 8,
wherein each of the one or more wells further includes a guide member that guides a flow of the culture solution discharged from the liquid passage portion.

10. The cell culture container according to claim 9,
wherein the guide member is provided on the outer circumferential surface of each of the one or more wells in a region closer to the lower end of the bottom portion than the liquid passage portion and extends in the opposite direction to the liquid passage portion.

11. A cell culture method, comprising:
preparing the cell culture container according to any one of claims 1 to 10;
immersing the cells retained in the one or more wells in the culture solution accommodated in the first container to culture the cells and cell masses obtained by aggregating the cells; and
observing the cell masses,
wherein the observing of the cell masses includes removing the second container from the first container, and the cell masses in the one or more wells are observed when some of the culture solution is discharged from the one or more wells through the liquid passage portion.

12. The cell culture method according to claim 11,
wherein the observing of the cell masses includes removing a liquid adhered to the outer circumference of the bottom portion of the second container after the second container is removed from the first container.

13. A method for manufacturing the cell culture container according to claim 1, comprising
manufacturing the second container by molding,
wherein the manufacturing of the second container includes
preparing a mold including an upper mold having a projection part having a shape corresponding to an outer shape of each of the one or more wells, a lower mold that is arranged facing the projection part and has a recess part having a shape corresponding to an outer shape of each of the one or more wells, and a core mold that penetrates the projection part and the recess part, and
curing a resin material poured into the mold to mold the second container including the one or more wells, and
wherein a hole as the liquid passage portion is formed with the core mold.

14. A method for manufacturing the cell culture container according to claim 1, comprising
manufacturing the second container by molding,
wherein the manufacturing of the second container includes
preparing a mold including an upper mold having a projection part having a shape corresponding to an outer shape of each of the one or more wells, and a lower mold that is arranged facing the projection part and has a recess part having a shape corresponding to an outer shape of each of the one or more wells, and
curing a resin material poured into the mold to mold the second container including the one or more wells,
wherein the upper mold has a protrusion that extends in a vertical direction, projects from the projection part, and is in contact with the recess part, and
wherein the protrusion forms a slit as the liquid passage portion.

15. A method for manufacturing the cell culture container according to claim 1, comprising:
molding a molded product exhibiting an outer shape of the one or more wells by molding; and
forming a hole as the liquid passage portion in the molded product by laser emission.
